# EUROPEAN PATENT APPLICATION

(11) **EP 4 545 012 A1**
(43) Date of publication of application: **30.04.2025**
(21) Application number: 23206361.0
(22) Date of filing: 27.10.2023
(51) Int. Cl.: A61B 8/00, A61B 8/08

(54) **ULTRASOUND SYSTEM AND METHOD**

(71) Applicant: Koninklijke Philips N.V., 5656 AG Eindhoven (NL)
(72) Inventor: SHULEPOV, Sergey Yuryevich, Eindhoven (NL); DIRKSEN, Peter, Eindhoven (NL); TIMMERMANS, Peter, 5656AG Eindhoven (NL); DE WILD, Nico Maris Adriaan, Eindhoven (NL); PHAM, Hoa T.M., Eindhoven (NL)
(74) Representative: Philips Intellectual Property & Standards

(57) **Abstract**

An ultrasound system comprising a wearable ultrasound device and a processing system. The wearable ultrasound device comprises an ultrasound transducer arrangement having an adjustable field of view. The processing system is configured to control the wearable ultrasound device to rotate, tilt or otherwise adjust the angle of the imaging plane generated by the ultrasound transducer arrangement, to obtain a plurality of 2D ultrasound images, each acquired at a differently-angled imaging plane. The plurality of 2D ultrasound images are processed to identify a target anatomical feature. A desired imaging plane for imaging the target anatomical feature is determined, and ultrasound imaging data at the desired imaging plane is acquired.

## Description

### FIELD OF THE INVENTION

The invention relates to the field of ultrasound imaging, and in particular, to ultrasound systems comprising a wearable ultrasound device.

### BACKGROUND OF THE INVENTION

Wearable ultrasound devices are becoming increasingly common for a variety of monitoring applications.

However, there are challenges in using wearable ultrasound devices in some ultrasound applications. For instance, in fetal monitoring, fetal movement can cause a change in position of an anatomical feature of interest relative to the ultrasound transducer. In a conventional fetal ultrasound examination, a skilled sonographer will adjust a position and/or orientation of the ultrasound probe used to perform the ultrasound examination; however, wearable ultrasound devices are generally used in the absence of a sonographer.

There is therefore a need for improvement in wearable ultrasound technology.

### SUMMARY OF THE INVENTION

The invention is defined by the claims.

According to examples in accordance with an aspect of the invention, there is provided an ultrasound system comprising: a wearable ultrasound device, comprising an ultrasound transducer arrangement, wherein the ultrasound transducer arrangement is capable of generating an imaging plane at a plurality of different angles; and a processing system in communication with the wearable ultrasound device, the processing system being configured to: control the wearable ultrasound device to sequentially acquire a first plurality of initial 2D ultrasound images, each at an imaging plane having a different angle with respect to one another; receive the first plurality of initial 2D ultrasound images; process the first plurality of initial 2D ultrasound images to identify a target anatomical feature in the first plurality of initial 2D ultrasound images; process the identification of the target anatomical feature to determine a first desired imaging plane for imaging the target anatomical feature; and control the wearable ultrasound device to acquire ultrasound imaging data at the first desired imaging plane.

This allows a target anatomical feature to be imaged by a wearable ultrasound device without requiring a skilled operator to position the wearable ultrasound device appropriately. By automatically identifying a target anatomical feature, an appropriate imaging plane for imaging the target anatomical feature from a current position of the wearable ultrasound device may be automatically determined.

The processing system may be configured to sequentially acquire the first plurality of initial 2D ultrasound images by rotating or tilting the imaging plane over a 3D volume. For instance, the processing system may be configured to rotate the imaging plane around a central imaging axis, or to tilt the imaging axis of the imaging plane with respect to the wearable ultrasound device.

In some examples, the processing system is further configured to: process the ultrasound imaging data acquired at the first desired imaging plane to determine a measure of quality of anatomical feature recognition for the target anatomical feature; in response to the measure of quality exceeding a predetermined quality threshold, control the wearable ultrasound device to continue acquiring ultrasound imaging data at the first desired imaging plane; and in response to the measure of quality failing to exceed the predetermined quality threshold: control the wearable ultrasound device to sequentially acquire a second plurality of initial 2D ultrasound images, each at an imaging plane having a different angle with respect to one another; receive the second plurality of initial 2D ultrasound images; process the second plurality of initial 2D ultrasound images to identify the target anatomical feature in the second plurality of initial 2D ultrasound images; process the identification of the target anatomical feature to determine a second desired imaging plane for imaging the target anatomical feature; and control the wearable ultrasound device to acquire ultrasound imaging data at the second desired imaging plane.

In this way, the target anatomical feature may be continuously tracked without requiring a skilled operator to adjust the wearable ultrasound device, by automatically adjusting the imaging plane of the wearable ultrasound device once a current imaging plane is no longer able to image the target anatomical feature at a sufficiently high quality. This allows the target anatomical feature to continue to be monitored following a change in the position of the target anatomical feature relative to the wearable ultrasound device (e.g. due to fetal movement, in the case of fetal ultrasound monitoring).

In some examples, the processing system may be configured to repeat the step of determining a measure of quality, and controlling the wearable ultrasound device according to the determined measure of quality, at predetermined intervals.

In some examples, the wearable ultrasound device is capable of acquiring ultrasound imaging data in at least two imaging modalities. This enables better discrimination of the target anatomical feature.

In some examples, the ultrasound transducer arrangement comprises a plurality of transducer elements arranged in a flat radial array. A flat radial array provides greater penetration depth at smaller imaging angles.

In some examples, the ultrasound transducer arrangement comprises a plurality of transducer elements arranged in a conical array. The use of a conical arrangement of transducer elements improves image quality by improving the point spread function.

In some examples, each transducer element extends in a different radial direction of the array. In other words, the plurality of transducer elements are arranged in a spoke-like arrangement.

In some examples, each transducer element overlaps at least one other transducer element in a radial direction of the array. The use of overlapping (or staggered) transducer elements reduces image artifacts, compared to a spoke-like arrangement, thus improving image quality.

In some examples, the plurality of transducer elements comprise wafer-based transducer elements. Wafer technology enables the transducer elements to be more easily arranged in the radial or conical array.

In some examples, the plurality of transducer elements comprise CMUT elements, each CMUT element being formed from a plurality of CMUT drums. The use of CMUT elements enables bias switching, allowing a variable elevation dimension and thus improving image quality.

In some examples, each CMUT drum in a same CMUT element has a different size. This enables an active area of the ultrasound transducer arrangement to be increased.

In some examples, the ultrasound transducer arrangement comprises a plurality of linear arrays, each lying in a differently-angled plane with respect to one another.

In other words, the angle of the imaging plane generated by the wearable ultrasound device may be controlled by controlling which of the plurality of linear arrays is used to generate the imaging plane.

The plurality of linear arrays may, for example, comprise at least 4 linear arrays. This would allow the wearable ultrasound device to cover an abdominal volume during fetal monitoring such that the fetal heart would always be at least partially within a field of view.

In some examples, the ultrasound transducer arrangement comprises a linear array configured to rotate, about an imaging axis of the linear array, with respect to the wearable ultrasound device.

In other words, the angle of the imaging plane generated by the wearable ultrasound device may be controlled by controlling a rotation of the transducer element.

In some examples, the ultrasound transducer arrangement comprises a linear array configured to tilt, wherein tilting the linear array changes an angle of an imaging axis of the linear array with respect to the wearable ultrasound device.

In other words, the angle of the imaging plane generated by the wearable ultrasound device may be controlled by controlling a tilt of the transducer element with respect to the wearable ultrasound device.

According to another aspect of the invention, there is provided a computer-implemented method comprising: controlling a wearable ultrasound device to sequentially acquire a first plurality of initial 2D ultrasound images, each acquired at an imaging plane having a different angle with respect to one another; receiving the first plurality of initial 2D ultrasound images; processing the first plurality of initial 2D ultrasound images to identify a target anatomical feature in the first plurality of initial 2D ultrasound images; processing the identification of the target anatomical feature to determine a first desired imaging plane for imaging the target anatomical feature; and controlling the wearable ultrasound device to acquire ultrasound imaging data at the first desired imaging plane.

There is also proposed a computer program product comprising computer program code means which, when executed on a computing device having a processing system, cause the processing system to perform all of the steps of the method according to claim 14.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates the general operation of an exemplary ultrasound system;
Fig. 2 illustrates an ultrasound system 200, according to an embodiment of the invention;
Fig. 3 illustrates an example ultrasound transducer arrangement that may be used in the wearable ultrasound device of the ultrasound system of Fig. 2;
Fig. 4 illustrates another example ultrasound transducer arrangement that may be used in the wearable ultrasound device of the ultrasound system of Fig. 2;
Fig. 5 illustrates another example ultrasound transducer arrangement that may be used in the wearable ultrasound device of the ultrasound system of Fig. 2;
Fig. 6 illustrates another example ultrasound transducer arrangement that may be used in the wearable ultrasound device of the ultrasound system of Fig. 2;
Fig. 7 illustrates a side view of a wearable ultrasound device that may be used in the ultrasound system of Fig. 2;
Fig. 8 illustrates a cross-sectional view of another wearable ultrasound device that may be used in the ultrasound system of Fig. 2;
Fig. 9 illustrates a cross-sectional view of another wearable ultrasound device that may be used in the ultrasound system of Fig. 2; and
Fig. 10 illustrates a computer-implemented method, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ultrasound system comprising a wearable ultrasound device and a processing system. The wearable ultrasound device comprises an ultrasound transducer arrangement having an adjustable field of view. The processing system is configured to control the wearable ultrasound device to rotate, tilt or otherwise adjust the angle of the imaging plane generated by the ultrasound transducer arrangement, to obtain a plurality of 2D ultrasound images, each acquired at a differently-angled imaging plane. The plurality of 2D ultrasound images are processed to identify a target anatomical feature. A desired imaging plane for imaging the target anatomical feature is determined, and ultrasound imaging data at the desired imaging plane is acquired.

Illustrative embodiments may, for example, be employed in ultrasound systems for fetal and/or abdominal monitoring.

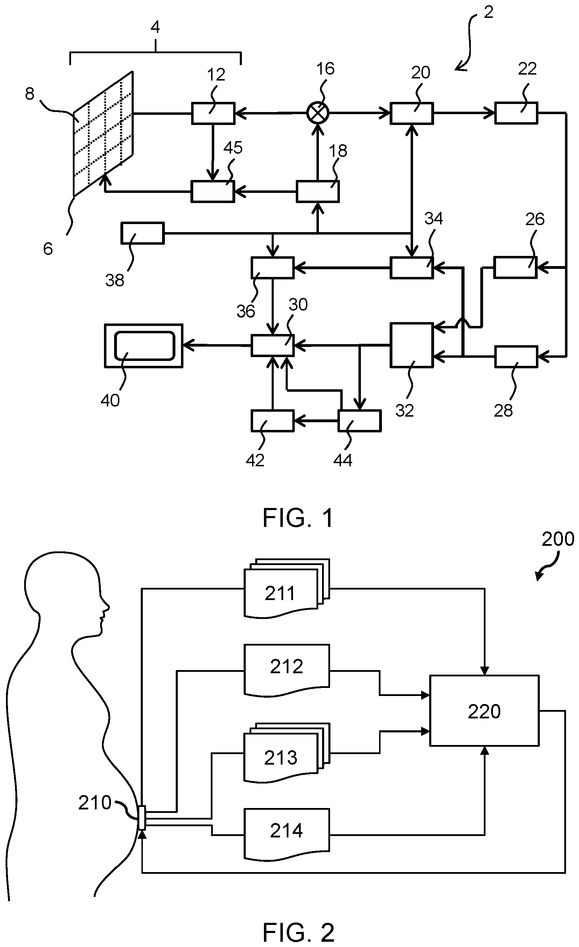

The general operation of an exemplary ultrasound system will first be described, with reference to Fig. 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission microbeamformer, and a main transmission beamformer.

The function of the microbeamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes, the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

Fig. 2 illustrates an ultrasound system 200, according to an embodiment of the invention. The ultrasound system comprises a wearable ultrasound device 210 and a processing system 220. The wearable ultrasound device may be an ultrasound patch, or any other kind of ultrasound device that may be worn by a subject for long periods of time (e.g. for several hours).

The wearable ultrasound device 210 comprises an ultrasound transducer arrangement that is capable of generating an imaging plane at a plurality of different angles. The ultrasound transducer arrangement may comprise any kind of transducer (e.g. wafer-based transducers, such as CMUTs or PMUTs, or conventional piezoelectric transducers). Various structures of the wearable ultrasound device and ultrasound transducer arrangement are envisaged, and are described in more detail below.

The imaging planes that can be generated by the wearable ultrasound device 210 are sufficient to allow an anatomical feature of interest to be imaged by at least one of the imaging planes at any point in time (assuming a correct positioning of the wearable ultrasound device on a subject). For instance, if the wearable ultrasound device is to be used to monitor a fetal heart, the wearable ultrasound device may be capable of generating an imaging plane having a field of view of at least 95°. The imaging plane generated by the wearable ultrasound device may, for example, be rotated 360° about a central imaging axis, or the imaging axis of the imaging plane may be tilted with respect to the wearable ultrasound device over a range of at least 40°, preferably at least 50° (for example, the imaging axis may be tilted by ±20-25° from an imaging axis perpendicular to the wearable ultrasound device).

Preferably, the wearable ultrasound device 210 is capable of acquiring ultrasound imaging data in at least two imaging modalities. For instance, the wearable ultrasound device may be capable of operating in a duplex mode (e.g. B-mode/M-mode and pulsed wave Doppler). In some examples, the wearable ultrasound device may be capable of operating in a triplex mode (e.g. B-mode/M-mode, pulsed wave Doppler and color Doppler/B-flow).

In Fig. 2, the wearable ultrasound device 210 is an ultrasound patch worn for the purpose of fetal monitoring. The ultrasound system 200 is particularly advantageous for fetal monitoring, because it allows an anatomical feature of interest of a fetus to be continuously imaged in spite of fetal movement, without requiring an expert sonographer to interpret imaging data acquired by the ultrasound system and manually adjust the wearable ultrasound device. However, as the skilled person will readily appreciate, the ultrasound system 200 is not limited to fetal monitoring applications, and may be used in any ultrasound monitoring system for which blood flow and/or tissue motion is relevant, such as abdominal monitoring.

The processing system 220 is in communication with the wearable ultrasound device 210. Although the processing system is illustrated as separate to the wearable ultrasound device in Fig. 2, in some examples, the processing system may be included in the wearable ultrasound device, and may be in communication with the wearable ultrasound device by a wired connection. In other examples, the processing system may be provided in one or more devices separate to the wearable ultrasound device, and may communicate wirelessly with the wearable ultrasound device. In yet other examples, parts of the processing system may be included in the wearable ultrasound device and other parts may be provided in one or more separate devices, and various parts of the processing system may communicate with one another wirelessly.

The processing system 220 is configured to control the wearable ultrasound device to sequentially acquire a first plurality of initial 2D ultrasound images 211. Each image of the first plurality of initial 2D ultrasound images 211 is acquired at a differently-angled imaging plane, by rotating the imaging plane generated by the ultrasound transducer arrangement. The imaging plane may be rotated about a central imaging axis and/or the angle of the imaging axis with respect to the wearable ultrasound device may be altered. In this way, a 3D volume containing a target anatomical feature is imaged.

The processing system 220 is configured to receive the first plurality of initial 2D ultrasound images acquired by the wearable ultrasound device 211, and to process the first plurality of initial 2D ultrasound images to identify a target anatomical feature. The target anatomical feature may be any anatomical feature which the wearable ultrasound device is being used to monitor. For instance, in fetal monitoring systems, the target anatomical feature may be a fetal heart, a fetal aorta or an umbilical cord.

Methods for identifying a target anatomical feature in an ultrasound image are well-known, and suitable techniques, including machine-learning and computer vision techniques, will be apparent to the skilled person. For example, European Patent Application No. EP 4,029,453 A1 describes a technique for determining a location of the heart of a fetus.

In some examples, the first plurality of initial 2D ultrasound images may be preprocessed before applying one or more anatomical feature recognition techniques, in order to improve an image quality. Suitable image quality improvement techniques, such as apodization, will be readily apparent to the skilled person.

Having identified the target anatomical feature in the first plurality of initial 2D ultrasound images, the processing system 220 is configured to process the identification of the target anatomical feature to determine a first desired imaging plane for imaging the target anatomical feature. For instance, if only one imaging plane that can be generated by the wearable ultrasound device contains the target anatomical feature, this imaging plane may be selected as the first desired imaging plane.

If several possible imaging planes contain the anatomical feature, the imaging plane providing the "best" view of the target anatomical feature may be selected as the first desired imaging plane. For example, if a machine-learning technique is used to identify the anatomical feature, a probability of recognition of the target anatomical feature may be determined for each of the initial 2D ultrasound images in which the target anatomical feature is identified, and the imaging plane of the initial 2D ultrasound image having the highest determined probability may be selected as the first desired imaging plane. If a computer vision algorithm is used, a quality metric may be determined for each of the initial 2D ultrasound images in which the target anatomical feature is identified (e.g. a signal-to-noise ratio, a contrast-to-noise ratio, a contrast resolution, a segmentation shape quality, etc.), and the imaging plane of the initial 2D ultrasound image having the highest quality metric may be selected as the first desired imaging plane.

The processing system 220 is then configured to control the wearable ultrasound device 210 to acquire ultrasound imaging data 212 at the first desired imaging plane. The ultrasound imaging data 212 may be used for monitoring the target anatomical feature; for example, the processing system may control the wearable ultrasound device to acquire the ultrasound imaging data 212 continuously or at regular predetermined intervals.

In some examples, a first imaging modality may be used to acquire the first plurality of initial 2D ultrasound images 211, and a second, different imaging modality may be used to acquire the ultrasound imaging data 212 at the desired imaging plane. For instance, B-mode imaging may be used to acquire the first plurality of initial 2D ultrasound images, and another imaging modality (e.g. M-mode or pulsed wave Doppler) may be used to acquire the ultrasound imaging data at the desired imaging plane, in order to monitor motion of the target anatomical feature.

In some examples, the processing system 220 may control the wearable ultrasound device 210 to acquire the ultrasound imaging data 212 at the first desired imaging plane at a particular depth range containing the target anatomical feature. By limiting the depth range at which the ultrasound imaging data 212 is acquired, clutter from other tissue motion, which may distort monitoring of the target anatomical feature, may be reduced.

In some examples, the processing system 220 may be further configured to dynamically adjust the imaging plane at which ultrasound imaging data is acquired to ensure that a current imaging plane is appropriate for imaging the target anatomical feature.

For instance, the processing system 220 may be configured to receive and process the ultrasound imaging data 212 acquired by the wearable ultrasound imaging device at the first desired imaging plane, in order to determine a measure of quality of anatomical feature recognition for the target anatomical feature.

Techniques for determining a measure of quality of anatomical feature recognition in ultrasound imaging data are well known. Suitable measures of quality of anatomical feature recognition include standard image quality metrics (signal-to-noise ratio, contrast resolution, etc.), a quality metric of anatomical feature segmentation (e.g. based on a confidence of region mapping), a scoring metric of a machine-learning algorithm used to (in cases where a machine-learning algorithm is used), a Doppler signal quality, quality metrics based on a comparison with data from an external sensor (e.g. IMU data may be mapped to the ultrasound imaging data), and quality metrics based on outlier detection.

Having determined the measure of quality, the processing system 220 may be configured to compare the measure of quality with a predetermined quality threshold. The value of the predetermined quality threshold may be set such that ultrasound imaging data for which the target anatomical feature cannot be recognized with a desired degree of confidence has a measure of quality below the predetermined quality threshold. The skilled person will appreciate that a suitable value for the predetermined quality threshold will therefore depend on the measure of quality of anatomical feature recognition used, and on the type of target anatomical feature.

In response to the measure of quality exceeding the predetermined quality threshold, the processing system 220 may be configured to control the wearable ultrasound device 210 to continue acquiring ultrasound imaging data 212 at the first desired imaging plane.

In response to the measure of quality failing to exceed the predetermined quality threshold, the processing system 220 may be configured to repeat the steps of identifying a desired imaging plane for imaging the target anatomical feature. In other words, the processing system 220 may control the wearable ultrasound device 210 to sequentially acquire a second plurality of initial 2D ultrasound images 213. As with the first plurality of initial 2D ultrasound images 211, each image of the second plurality of initial 2D ultrasound images is acquired at an imaging plane having a different angle with respect to one another.

The processing system 220 may then be configured to receive and process the second plurality of initial 2D ultrasound images 213 as described above with respect to the first plurality of initial 2D ultrasound images, in order to identify the target anatomical feature in the second plurality of initial 2D ultrasound images.

Having identified the target anatomical feature in the second plurality of initial ultrasound images 213, the processing system 220 may be configured to process the (new) identification of the target anatomical feature to determine a second desired imaging plane for imaging the target anatomical feature. The processing system may then be configured to control the wearable ultrasound device 210 to acquire ultrasound imaging data 214 at the second desired imaging plane.

In some examples, the processing system 220 may be configured to determine the measure of quality at regular, predetermined intervals throughout use of the wearable ultrasound device 210, determining a new desired imaging plane for imaging the target anatomical feature as required (i.e. once the measure of quality fails to exceed the predetermined quality threshold). In this way, the target anatomical feature may be automatically tracked and imaged throughout use of the wearable ultrasound device.

In some examples, the processing system 220 may be configured to determine the measure of quality in response to a determination that a measure of movement of the target anatomical feature (e.g. velocity, displacement relative to the wearable ultrasound device, etc.) exceeds a predetermined movement threshold. In other words, the processing system 220 may process the ultrasound imaging data acquired at a current desired imaging plane to track movement of the target anatomical feature, and determine the measure of quality, adjusting the imaging plane as required, in response to a sufficient amount of movement of the target anatomical feature.

As described above, the wearable ultrasound device 210 comprises an ultrasound transducer arrangement capable of generating an imaging plane at a plurality of different angles. In some examples, the ultrasound transducer arrangement may be capable of generating an imaging plane at a plurality of different angles having a same central imaging axis (i.e. the imaging plane may be adjusted by rotating the imaging plane about the central imaging axis). Additionally or alternatively, the ultrasound transducer arrangement may be capable of generating imaging planes having a central imaging axis at a different angle (i.e. a different angle with respect to the wearable ultrasound device).

The imaging plane generated by the ultrasound transducer arrangement may be controlled electronically, e.g. by controlling which transducer elements (or which arrays of transducer elements) are used to generate the imaging plane.

For instance, the ultrasound transducer arrangement may comprise a plurality of transducer elements arranged in an array to enable the generation of differently-angled imaging planes depending on which transducer elements are used to generate the imaging plane. The imaging plane generated by such an ultrasound transducer arrangement may be adjusted by rotating the imaging plane about a central imaging axis. For example, to acquire a plurality of initial 2D ultrasound images, the imaging plane may be rotated 360° about the imaging axis, and 2D ultrasound images may be acquired at regular intervals. In this way, a volume containing the target anatomical feature may be interrogated to identify the target anatomical feature.

In some examples, the ultrasound transducer arrangement may comprise a plurality of transducer elements arranged in a flat radial array (i.e. with the transducer elements lying in a same plane). The flat radial array may be a conventional 2D array, or may have a ring structure. A ring structure may be preferred for use in a wearable ultrasound device, as such a structure enables a wide field of view while requiring fewer channels.

An ultrasound transducer arrangement comprising a flat radial array of transducer elements may be achieved using any kind of transducer technology. However, wafer-based technology (e.g. CMUT or PMUT) may be preferred, as this allows the assembly of transducer elements in arrangements that improve the imaging performance of the ultrasound transducer arrangement.

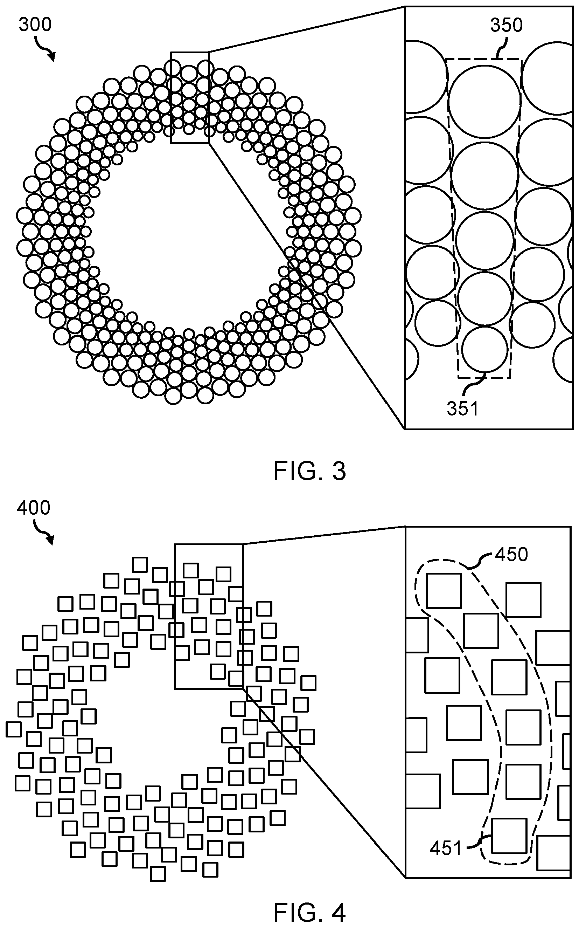

In particular, CMUT technology enables the assembly of transducer elements of a wide variety of shapes, by connecting individual CMUT drums into transducer elements having a desired shape, for example, to produce spoke-like arrangements, spiral arrangements, sunflower arrangements, sparse arrangements, etc. Examples of ultrasound transducer arrays that may be achieved using CMUT technology are illustrated in Figs 3 to 6.

Further, the use of an ultrasound transducer arrangement comprising transducer elements each formed from a plurality of CMUT drums enables an adjustable focal region of the ultrasound transducer arrangement, by using a switchable bias on the CMUT transducer elements. For instance, each transducer element may comprise one or more outer CMUT drums, further from the center of the array, and one or more inner CMUT drums, closer to the center of the array. A biasing voltage may be applied to the one or more outer CMUT drums of each transducer element where a greater penetration depth is required, and to the one or more inner CMUT drums where the desired penetration is less deep.

Fig. 3 illustrates an example ultrasound transducer arrangement 300 that may be used in the wearable ultrasound device 210. The ultrasound transducer arrangement 300 is a flat radial array having a ring structure. In the ultrasound transducer arrangement 300 of Fig. 3, each transducer element 350 extends in a different radial direction of the array, forming a spoke-like arrangement of transducer elements.

Each transducer element 350 is a CMUT element, formed from a plurality of CMUT drums 351. In order to increase an active area of the ultrasound transducer arrangement, each CMUT drum in a transducer element of the ultrasound transducer arrangement 300 has a different size but a same or similar (e.g. ±10%) central frequency. In particular, the size of the CMUT drums in each transducer element increases with distance from the center of the array. This allows the transducer elements to be provided closer to one another.

In some examples, rather than each transducer element of a radial array extending in a different radial direction, each transducer element may overlap at least one other transducer element in a radial direction of the array. This reduces the image artifacts found in ultrasound imaging data acquired by an array having a spoke-like arrangement of transducer elements, thus enabling improved identification of a target anatomical feature.

Fig. 4 illustrates an example ultrasound transducer arrangement 400 that may be used in the wearable ultrasound device 210. The ultrasound transducer arrangement 400 is a flat radial array in which the transducer elements 450 have a spiral arrangement. Each transducer element is a CMUT element formed from a plurality of CMUT drums 451.

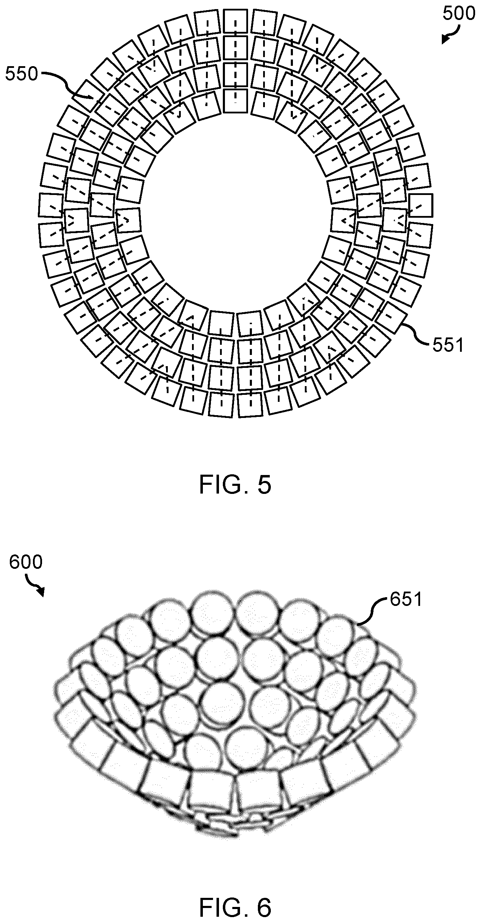

Fig. 5 illustrates an example ultrasound transducer arrangement 500 that may be used in the wearable ultrasound device 210. The ultrasound transducer 500 is a flat radial array of transducer elements. Each transducer element is a CMUT element formed from a plurality of CMUT drums 551. The dotted lines 550 connecting the CMUT drums illustrate how the CMUT drums are connected into CMUT elements. In the ultrasound transducer arrangement 500 of Fig. 5, the CMUT drums are provided in a staggered arrangement, such that the CMUT drums overlap in a radial direction.

In Figs 3 to 5, the ultrasound transducer arrangements are formed of a single ring of CMUT elements; however, the skilled person will appreciate that the CMUT drums may be connected into transducer elements in different ways to those shown in the Figures. For instance, the CMUT drums may be connected into elements to provide more than one ring of CMUT elements (e.g. each element of the arrangements shown in Figs 3 to 5 may instead be provided as two separate elements, resulting in an inner ring and outer ring of elements). In other examples, each square of the arrangements illustrated in Figs 4 and 5, instead of representing a single CMUT drum, may represent a transducer element formed from a plurality of CMUT drums.

In some examples, the ultrasound transducer arrangement may comprise a plurality of transducer elements provided in differently-angled planes, e.g. transducer elements arranged in a conical array. This improves imaging quality, particularly at large scanning angles, by improving the point spread function. Preferably, the conical array has an angle of 10-15° with respect to a central axis of the conical array.

As with the flat radial arrays described above, various shapes of transducer elements (e.g. spoke-like arrangements, overlapping/staggered arrangements, etc.) may be used in a conical array. Preferably, wafer-based technology may be used to provide the conical array. However, the skilled person will readily appreciate that a conical array may be formed using any kind of transducer technology, including conventional piezoelectric transducer (e.g. by securing piezoelectric transducer elements to a flexible foil, which is in turn secured to a conical backing).

Fig. 6 illustrates an example ultrasound transducer arrangement 600 that may be used in the wearable ultrasound device 210. The ultrasound transducer 600 is a conical array of transducer elements. Each transducer element is a CMUT element formed from a plurality of CMUT drums 651. As described above with respect to flat radial arrays, the CMUT drums may be connected into transducer elements in a variety of ways (e.g. a single conical ring with elements that overlap one another, a multiple ring arrangement, etc.). In some examples, each cylinder of the arrangement illustrated in Fig. 6 may represent, rather than a single CMUT drum, a transducer element formed from a plurality of CMUT drums.

In some examples, the ultrasound transducer arrangement may comprise a plurality of linear arrays, each lying in a differently-angled plane with respect to one another. The angle of the imaging plane generated by the ultrasound transducer arrangement may be controlled by controlling which linear array is used to generate the imaging plane.

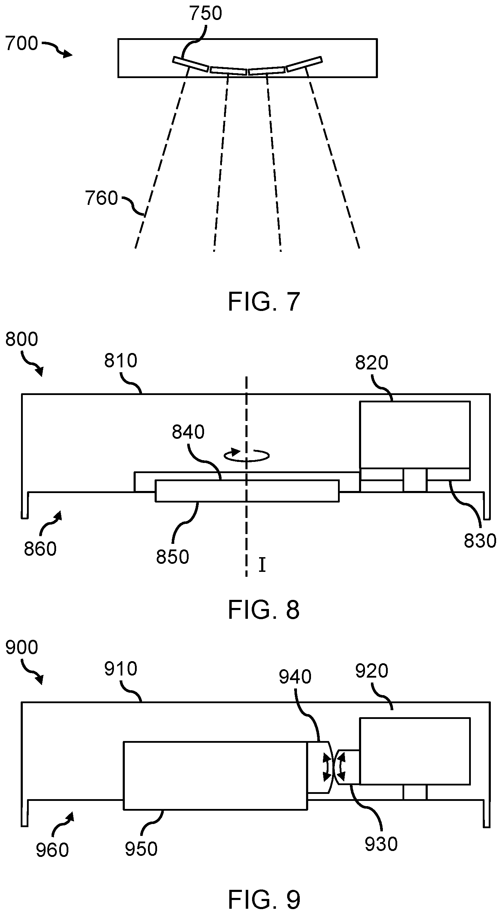

Fig. 7 illustrates a side view of a wearable ultrasound device 700 that may be used in the ultrasound system 200. The wearable ultrasound device 700 comprises an ultrasound transducer arrangement comprising four linear arrays 750, each provided at a different angle to one another. The imaging plane 760 generated by each linear array of the ultrasound transducer arrangement thus has a different angle with respect to the wearable ultrasound device 700.

Four linear arrays are sufficient to cover an abdominal volume such that, when used for abdominal monitoring, a fetal heart would always be at least partially imaged by the imaging plane produced by at least one of the linear arrays. Where four linear arrays are used, the imaging planes generated by the arrays may have an angle of 10-20°, preferably 15-20°, between each imaging plane (providing a total range of 30-60°, preferably 45-60°, across all imaging planes). The skilled person will readily appreciate that a greater number of linear arrays (with imaging planes at smaller angles with respect to one another) will be used, and that more arrays will provide improved monitoring of a target anatomical feature.

The linear arrays may be conventional linear arrays (e.g. comprising piezoelectric transducer elements) or may comprise wafer-based transducer elements. Preferably, CMUT elements are used, in order to allow bias switching (allowing the imaging plane to be moved to different positions with respect to the linear array by controlling which CMUT drums have a voltage applied to them).

For illustrative purposes, the linear arrays 750 have been provided in a single row in Fig. 7. However, the skilled person will appreciate that other arrangements may be used; for example, the linear arrays may be provided in a grid arrangement.

Figs 3 to 7 illustrates ultrasound transducer arrangements for which the angle of an imaging plane generated by the ultrasound transducer arrangement is controlled by controlling which transducer element(s) or array(s) are used to generate the imaging plane. In other examples, the imaging plane generated by the ultrasound transducer arrangement may be controlled by mechanically adjusting the ultrasound transducer arrangement.

Fig. 8 illustrates a cross-sectional view of a wearable ultrasound device 800 that may be used in the ultrasound system 200. The wearable ultrasound device 800 comprises a housing 810 that contains a motor 820, first and second gears 830 and 840, and ultrasound transducer arrangement 850 comprising a linear array.

The motor 820 rotates the first gear 830, which engages with the second gear 840, causing the second gear to rotate. The ultrasound transducer arrangement 850 is attached to the second gear; in this way, rotation of the motor causes the ultrasound transducer arrangement to rotate about imaging axis I, allowing an angle of the imaging plane to be adjusted.

The ultrasound transducer arrangement 850 is recessed within a cavity 860 of the wearable ultrasound device. The cavity may be filled with a coupling medium (e.g. a coupling liquid or a soft gel coupling layer) to provide acoustic coupling between the ultrasound transducer arrangement and a subject wearing the wearable ultrasound device.

The ultrasound transducer arrangement 850 may be connected to a beamformer (not shown in Fig. 8) by a connector that does not restrict rotation of the ultrasound transducer arrangement, e.g. by a length of wire that is longer than the distance between the ultrasound transducer arrangement and the beamformer and is folded or coiled within the housing 810.

Fig. 9 illustrates a cross-sectional view of a wearable ultrasound device 900 that may be used in the ultrasound system 200. The wearable ultrasound device 900 comprises a housing 910 that contains a motor 920, first and second driving elements 930 and 940, and ultrasound transducer arrangement 950 comprising a linear array. The first driving element is attached to the motor, while the second driving element is attached to the ultrasound transducer arrangement.

Vibrations of motor 920 cause the first driving element 930 and second driving element to move with respect to one another, which in turn causes the ultrasound transducer arrangement to tilt, i.e. to rotate with respect to the housing of the wearable ultrasound device such that an angle of the imaging axis of the linear array with respect to the housing is adjusted.

As with the wearable ultrasound device 800, the ultrasound transducer arrangement 950 is recessed within a cavity 960 of the wearable ultrasound device 900. The cavity may be filled with a coupling medium. A coupling liquid would enable the ultrasound transducer arrangement to tilt across a greater angle range than a soft gel coupling would.

As the skilled person will readily appreciate that configurations other than those illustrated in Figs 8 and 9 may be used to provide a mechanically-adjustable ultrasound transducer arrangement.

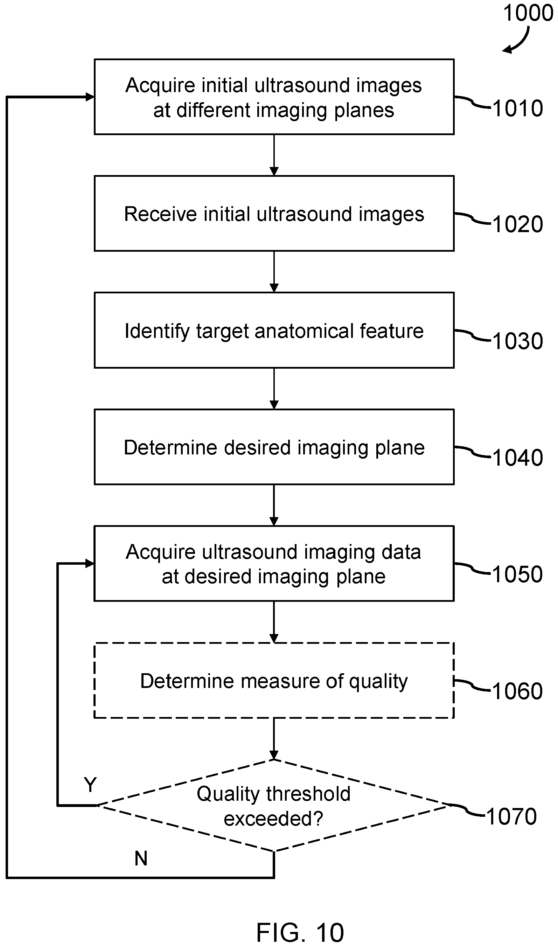

Fig. 10 illustrates a computer-implemented method 1000, according to an embodiment of the invention.

The computer-implemented method 1000 begins at step 1010, at which a wearable ultrasound device is controlled to sequentially acquire a first plurality of initial 2D ultrasound images, each acquired at an imaging plane having a different angle with respect to one another.

At step 1020, the first plurality of initial 2D ultrasound images are received (i.e. by a processing system performing the method 1000).

At step 1030, the first plurality of initial 2D ultrasound images are processed to identify a target anatomical feature in the first plurality of initial 2D ultrasound images.

At step 1040, the identification of the target anatomical feature is processed to determine a first desired imaging plane for imaging the target anatomical feature.

At step 1050, the wearable ultrasound device is controlled to acquire ultrasound imaging data at the first desired imaging plane.

In some examples, the computer-implemented method may further comprise a step 1060, at which the ultrasound imaging data acquired at the first desired imaging plane is processed to determine a measure of quality of anatomical feature recognition for the target anatomical feature, and a step 1070, at which the determined measure of quality is compared to a predetermined quality threshold.

In response to the measure of quality exceeding the predetermined quality threshold, the wearable ultrasound device may continue to be controlled to acquire ultrasound imaging data at the first desired imaging plane.

In response to the measure of quality failing to exceed the predetermined quality threshold, steps 1010 to 1050 may be repeated, in order to acquire ultrasound imaging data at a new desired imaging plane.

The step 1060 of determining a measure of quality may be repeated at predetermined intervals.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An ultrasound system (200) comprising:
A wearable ultrasound device (210, 700, 800, 900), comprising an ultrasound transducer arrangement (300, 400, 500, 600, 850, 950), wherein the ultrasound transducer arrangement is capable of generating an imaging plane at a plurality of different angles; and
a processing system (220) in communication with the wearable ultrasound device, the processing system being configured to:
control the wearable ultrasound device to sequentially acquire a first plurality of initial 2D ultrasound images (211), each at an imaging plane having a different angle with respect to one another;
receive the first plurality of initial 2D ultrasound images;
process the first plurality of initial 2D ultrasound images to identify a target anatomical feature in the first plurality of initial 2D ultrasound images;
process the identification of the target anatomical feature to determine a first desired imaging plane for imaging the target anatomical feature; and
control the wearable ultrasound device to acquire ultrasound imaging data (212) at the first desired imaging plane.

2. The ultrasound system (200) of claim 1, wherein the processing system is further configured to:
process the ultrasound imaging data (212) acquired at the first desired imaging plane to determine a measure of quality of anatomical feature recognition for the target anatomical feature;
in response to the measure of quality exceeding a predetermined quality threshold, control the wearable ultrasound device (210, 700, 800, 900) to continue acquiring ultrasound imaging data at the first desired imaging plane; and
in response to the measure of quality failing to exceed the predetermined quality threshold:
control the wearable ultrasound device to sequentially acquire a second plurality of initial 2D ultrasound images (213), each at an imaging plane having a different angle with respect to one another;
receive the second plurality of initial 2D ultrasound images;
process the second plurality of initial 2D ultrasound images to identify the target anatomical feature in the second plurality of initial 2D ultrasound images;
process the identification of the target anatomical feature to determine a second desired imaging plane for imaging the target anatomical feature; and
control the wearable ultrasound device to acquire ultrasound imaging data (214) at the second desired imaging plane.

3. The ultrasound system (200) of claim 2, wherein the processing system is configured to repeat the step of determining a measure of quality, and controlling the wearable ultrasound device (210, 700, 800, 900) according to the determined measure of quality, at predetermined intervals.

4. The ultrasound system (200) of any of claims 1 to 3, wherein the ultrasound transducer arrangement (300, 400, 500) comprises a plurality of transducer elements (350, 450, 550) arranged in a flat radial array.

5. The ultrasound system (200) of any of claims 1 to 3, wherein the ultrasound transducer arrangement (600) comprises a plurality of transducer elements arranged in a conical array.

6. The ultrasound system (200) of claim 4 or 5, wherein each transducer element (350) extends in a different radial direction of the array.

7. The ultrasound system (200) of claim 4 or 5, wherein each transducer element (450, 550) overlaps at least one other transducer element in a radial direction of the array.

8. The ultrasound system (200) of any of claims 4 to 7, wherein the plurality of transducer elements (350, 450, 550) comprise wafer-based transducer elements.

9. The ultrasound system (200) of claim 8, wherein the plurality of transducer elements (350, 450, 550) comprise CMUT elements, each CMUT element being formed from a plurality of CMUT drums (351, 451, 551, 651).

10. The ultrasound system (200) of claim 9, wherein each CMUT drum (351) in a same CMUT element (350) has a different size.

11. The ultrasound system (200) of any of claims 1 to 3, wherein the ultrasound transducer arrangement comprises a plurality of linear arrays (750), each lying in a differently-angled plane with respect to one another.

12. The ultrasound system (200) of any of claims 1 to 3, wherein the ultrasound transducer arrangement (850) comprises a linear array configured to rotate, about an imaging axis (I) of the linear array, with respect to the wearable ultrasound device (800).

13. The ultrasound system of any of claims 1 to 3, wherein the ultrasound transducer arrangement (950) comprises a linear array configured to tilt, wherein tilting the linear array changes an angle of an imaging axis of the linear array with respect to the wearable ultrasound device (900).

14. A computer-implemented method (1000) comprising:
controlling a wearable ultrasound device (210, 700, 800, 900) to sequentially acquire a first plurality of initial 2D ultrasound images (211), each acquired at an imaging plane having a different angle with respect to one another;
receiving the first plurality of initial 2D ultrasound images;
processing the first plurality of initial 2D ultrasound images to identify a target anatomical feature in the first plurality of initial 2D ultrasound images;
processing the identification of the target anatomical feature to determine a first desired imaging plane for imaging the target anatomical feature; and
controlling the wearable ultrasound device to acquire ultrasound imaging data (212) at the first desired imaging plane.

15. A computer program product comprising computer program code means which, when executed on a computing device having a processing system, cause the processing system to perform all of the steps of the method (1000) according to claim 14.
